# EUROPEAN PATENT SPECIFICATION

(11) **EP 3 346 989 B1**
(45) Date of publication and mention of the grant of the patent: **30.12.2020**
(21) Application number: 15903759.7
(22) Date of filing: 21.09.2015
(51) Int. Cl.: A61K 9/127, A61K 31/436, A61P 35/00, A61P 35/02

(54) **STABLE LIPOSOMAL FORMULATIONS OF RAPAMYCIN AND RAPAMYCIN DERIVATIVES FOR TREATING CANCER**
STABILE LIPOSOMALE FORMULIERUNGEN VON RAPAMYCIN UND RAPAMYCINDERIVATEN ZUR BEHANDLUNG VON KREBS
FORMULATIONS LIPOSOMALES STABLES DE RAPAMYCINE ET DE DÉRIVÉS DE RAPAMYCINE POUR LE TRAITEMENT DU CANCER

(30) Priority: 09.09.2015 US 201514849100
(43) Date of publication of application: 18.07.2018
(73) Proprietor: Manli International Ltd., 558445 Singapore (SG)
(72) Inventor: SHI, Zhiwei, Irvine, CA 92616 (US); SU, Shih-horng, Irvine, CA 92606 (US); YU, Ting-bin, Irvine, CA 92620 (US)
(74) Representative: Cabinet Becker et Associés
(86) International application number: PCT/US2015/051206
(87) International publication number: WO 2017/044135

(56) References cited:
- WO-A1-2012/118376
- WO-A2-2014/121211
- WO-A2-2015/068020
- US-A1- 2011 244 028
- US-A1- 2013 337 051
- US-A1- 2014 154 305
- US-A1- 2014 154 305
- US-A1- 2015 079 155

## Description

### BACKGROUND

### Field

The application relates to the field of anti-cancer drugs, in particular, methods for loading hydrophobic anti-cancer drugs into liposomes and for treatment of cancers with the liposomes.

### Background

Rapamycin, also known as sirolimus, is a macrolide antibiotic initially developed for use as an immune suppressor for transplant patients. Subsequently, it was used as a drug coating for coronary artery stents, where it functions to reduce restenosis following angioplasty by inhibiting smooth muscle cell proliferation.

Sirolimus and derivatives of this drug have also been found to be effective for treating certain cancers. For example, sirolimus has anti-tumor activity. See US Patent 4,885,171. Everolimus, the 40-0-(2-hydroxyethyl) derivative of sirolimus, has been approved for treating advanced kidney cancer, advanced hormone receptor-positive/HER2-negative breast cancer, and pancreatic neuroendocrine tumors.

It has been demonstrated that umirolimus i.e., 40-alkoxyalkyl-rapamycin, and umirolimus-loaded polymer micelles can both inhibit cancer cell growth *in vitro* and the micelles are effective for slowing the growth of experimental tumors *in vivo.* See US Patent Application Publication 2014/0154305. The polymer micelle encapsulation of umirolimus significantly improves the solubility and stability of this drug and results in its sustained delivery.

As an alternative to polymer micelles, liposomes have been employed to improve drug delivery of sirolimus and its derivatives. For example, sirolimus, everolimus, and tacrolimus have been encapsulated in liposomes using two passive loading methods, namely, thin-film hydration and ethanol injection. However, due to low solubility and hydrophobicity of these drugs, the amount of drug encapsulated and drug encapsulation efficiency is particularly low, i.e., < 0.5 mg/mL and < 90%, respectively. Drug leakage from the liposomes also occurs with passive loading techniques.

A remote film loading technique that requires steps of drug dissolution and solvent removal has been used to entrap sirolimus into pre-formed liposomes. Although the method results in high drug encapsulation efficiency, there are potential risks to drug stability during the loading procedure.

Thus, the need exists for developing new liposomal loading methods for hydrophobic drugs to improve drug content, drug to lipid ratio, and drug encapsulation efficiency. Such methods should form drug-loaded liposomes having a higher therapeutic index as compared to existing liposomes, particularly for umirolimus and other rapamycin-derivatives.

### SUMMARY

To satisfy this need, a stable liposomal formulation for treating cancer is provided. The stable formulation includes a liposome that contains at least one lipid bilayer formed of one or more phosphatidylcholine selected from palmitoyloleoylphosphatidylcholine (POPC), dimyristoylphosphatidylcholine (DMPC), and dioleoylphosphatidylcholine (DOPC). The liposome has a diameter of 50 nm to 2 µm and is free of cholesterol. Encapsulated in the liposome is an anti-cancer drug selected from sirolimus, umirolimus, and everolimus.

Also provided is a method for loading a hydrophobic drug into liposomes. The method includes the steps of (i) obtaining cholesterol-free liposomes having at least one lipid bilayer, (ii) adding the cholesterol-free liposomes to an aqueous solution to form a suspension such that there is substantially no transmembrane potential across the lipid bilayer, (iii) adding a hydrophobic drug in the absence of a solubility enhancer to the suspension to form a mixture, and (iv) stirring the mixture for 4 to 48 hours at room temperature. The method results in loading of at least 80% of the hydrophobic drug into the liposomes. In an embodiment, the method consists of the steps set forth in this paragraph.

Additionally, a method for preparing a hydrophobic drug encapsulated in a cholesterol-free liposome is disclosed. The method is carried out by (i) suspending one or more of POPC, DMPC, and DOPC in an aqueous buffer to form a lipid suspension, (ii) stirring the lipid suspension for at least 30 minutes at room temperature to form multilamellar vesicles (MLVs), (iii) extruding the MLVs to form large unilamellar vesicles (LUVs) having a diameter of 50 nm to 2 µm, (iv) adding the LUVs to an aqueous solution to form a suspension such that there is substantially no transmembrane potential across the LUVs, (v) adding a hydrophobic drug to the suspension in the absence of a solubility enhancer to form a mixture, (vi) stirring the mixture for 4 to 48 hours at room temperature to form a drug-loaded liposome suspension, and (vii) filtering the drug-loaded liposome suspension to remove unencapsulated hydrophobic drug. The method allows for encapsulation of at least 80% of the added hydrophobic drug. In another embodiment, the method consists of the steps set forth in this paragraph.

A method for treating cancer is also disclosed. The method requires the steps of administering to a subject in need thereof an effective amount of the stable liposomal formulations described above. The effective amount is sufficient to inhibit growth of cancer cells in the subject.

The details of one or more embodiments of the invention are set forth in the description, in the drawings, and in the examples below. Other features, objects, and advantages of the invention will be apparent from the detailed description, the drawings, and also from the claims.

### BRIEF DESCRIPTION OF THE DRAWINGS

The invention description below refers to the accompanying drawings, of which:
Fig. 1 is a plot of the *in vitro* drug release profile of umirolimus (URL) and liposomal umirolimus (LipoURL);
Fig. 2 is a plot of the *in vitro* drug release profile of everolimus (ERL) and liposomal everolimus (LipoERL); and
Fig. 3 is a plot of the *in vitro* drug release profile of sirolimus (SRL), and liposomal sirolimus (LipoSRL).

### DETAILED DESCRIPTION

As mentioned above, a stable liposomal formulation for treating cancer is disclosed. The liposomes in the formulation contain at least one lipid bilayer formed of a phosphatidylcholine selected from POPC, DMPC, and DOPC, or mixtures of these three phosphatidylcholines, and are free of cholesterol. In a particular aspect, the liposomes contain one lipid bilayer. In another aspect, the liposomes contain only POPC, DMPC, or DOPC. In a preferred embodiment, the liposomes contain only POPC.

Alternatively, the lipid bilayer of the liposomes includes the phosphatidylcholine together with a phospholipid conjugated to a polyethylene glycol (PEG) moiety. The PEG-conjugated phospholipid can be 1,2-distearoyl-sn-glycero-3-phosphoethanolamine-N-[amino(PEG)](DSPE-PEG); 1,2-dioleoyl-sn-glycero-3-phosphoethanolamine-N-[methoxy(PEG)](DOPE-PEG); 1,2-dipalmitoyl-sn-glycero-3-phosphoethanolamine-N-[methoxy(PEG)](DPPE-PEG); 1,2-dimyristoyl-sn-glycero-3-phosphoethanolamine-N-[methoxy(PEG)](DMPE-PEG); or mixtures thereof. In a particular embodiment, the PEG-conjugated phospholipid is DSPE-PEG.

When the liposome includes both the phosphatidylcholine and the PEG-conjugated phospholipid, the weight ratio between them can be 5:1 to 100:1, e.g., 5:1, 7.5:1, 10:1, 15:1, 20:1, 30:1, 40:1, 50:1, 60:1, 70:1, 80:1, 90:1, and 100:1. A preferred ratio is 10:1.

The molecular weight of the PEG moiety that is conjugated to the phospholipid can be 150 to 3000 g/mol, e.g., 150, 200, 250, 300, 350, 500, 750, 1000, 1250 1500, 1750, 2000, 2250, 2500, 2750, 3000 g/mol. In a preferred embodiment, the molecular weight of the PEG moiety is 2000 g/mol.

The liposomes can have a diameter of 50 nm to 2 µm (e.g., 50 nm, 100 nm, 150 nm, 200 nm, 250 nm, 500 nm, 1 µm, 1.5 µm, and 2 µm). In an embodiment, the liposomes have a diameter of 50 nm to 500 nm. In a preferred embodiment, the diameter is 100 nm.

The liposomes in the stable liposomal formulation encapsulate a hydrophobic drug for treating cancer. The hydrophobic drug can be an anti-proliferative drug, e.g., sirolimus, umirolimus, or everolimus. In a particular formulation, the hydrophobic drug is umirolimus.

The weight ratio between the hydrophobic drug and the phosphatidylcholine component of the liposomes can be 1:5 to 1:100 (e.g., 1:5, 1:10, 1:15, 1:20, 1:25, 1:30, 1:35, 1:40, 1:45, 1:50, 1:75, and 1:100). In an embodiment, the drug to phosphatidylcholine weight ratio is 1:10. In another particular embodiment, the weight ratio is 1:20.

The concentration of the hydrophobic drug in the stable liposomal formulation can be 0.01 mg/mL to 10 mg/mL. For example, the drug concentration in the formulation can be 0.01 mg/mL, 0.05 mg/mL, 0.5 mg/mL, 1.0 mg/mL, 2.5 mg/mL, 5.0 mg/mL, and 10 mg/mL. In a particular embodiment, the drug concentration is 1 mg/mL.

Additionally, the stable liposomal formulation can have a pH of 6.0 to 8.0. In a preferred embodiment, the pH is 7.4.

In a particular aspect, the stable liposomal formulation for treating cancer includes liposomes formed only of POPC and DSPE-PEG 2000, the liposomes having umirolimus encapsulated therein. The liposomes are cholesterol-free, have a diameter of about 100 nm, and the weight ratio between the umirolimus and the POPC is 1:20. This specific formulation contains 1 mg/mL of umirolimus and has a pH of 7.4.

The stable liposomal formulation also improves the stability of the hydrophobic drug in solution. For example, the hydrophobic drug in the formulation can be stable for 7-14 days when stored at 5 °C as compared to the drug in an aqueous suspension. In this context, stability is defined as a loss of no more than 5 % of the starting amount of drug in the formulation.

Advantageously, the hydrophobic drug can be released from the liposomes in the formulation over an extended period of time after administration. That is to say, the formulation is a sustained release formulation. For example, the hydrophobic drug can be released from the liposomes after administration of the formulation continuously over a period of up to 3 months, e.g., 7, 14, 21 days and 1, 2, and 3 months.

Also mentioned above, a method for loading a hydrophobic drug into liposomes is provided. The method is an improved remote film loading technique in which the liposomes are obtained first and the hydrophobic drug is then loaded into the liposomes.

The liposomes for use in the loading method are cholesterol-free, have at least one lipid bilayer that contains one or more of POPC, DMPC, and DOPC, and have a diameter of 50 nm to 2 µm. In an embodiment, the liposomes contain only POPC.

Alternatively, the cholesterol-free liposomes have at least one lipid bilayer that contains one or more of POPC, DMPC, and DOPC and also contains a PEG-conjugated phospholipid selected from DSPE-PEG, DOPE-PEG, DPPE-PEG, and DMPE-PEG.

The molecular weight of the PEG moiety that is conjugated to the phospholipid can be 150 to 3000 g/mol, e.g., 150, 200, 250, 300, 350, 500, 750, 1000, 1250 1500, 1750, 2000, 2250, 2500, 2750, 3000 g/mol. In a preferred embodiment, the molecular weight of the PEG moiety is 2000 g/mol.

In a particular aspect, the liposomes contain only POPC. In another embodiment, the liposomes contain only POPC and DSPE-PEG2000.

The liposomes can be obtained by forming MLVs that contain one or more of POPC, DMPC, DOPC, and, optionally, DSPE-PEG2000, and extruding the MLVs to obtain cholesterol-free liposomes having a diameter of 50 nm to 2 µm. More specifically, one or more of POPC, DMPC, DOPC, and, optionally, DSPE-PEG2000 are suspended in an aqueous buffer to form a lipid suspension, the suspension is stirred for at least 30 minutes at room temperature to form MLVs.

The MLVs are converted into large unilamellar vesicles (LUVs) by an extrusion process. For example, the MLVs can be extruded through a 3-layered polycarbonate filter from 3 to 20 times. In a preferred extrusion process, the MLVs are extruded 10 times.

The polycarbonate filter can have a pore size ranging from 50 nm to 200 nm. In a particular embodiment, the pore size is 100 nm. Again, the resulting LUVs, i.e., liposomes, can have a diameter of 50 nm to 2 µm.

The cholesterol-free liposomes described above are then added to an aqueous solution to form a suspension. Importantly, the aqueous solution employed should be the same solution or similar to that used for producing the cholesterol-free liposomes such that there is substantially no transmembrane potential across the lipid bilayer of the liposomes. For example, the ionic strength, pH, and osmolarity should be closely matched such that there is no ionic gradient, pH gradient, or osmotic gradient across the liposomal membrane. This can be ensured, e.g., by using PBS to form the cholesterol-free liposomes and also diluting them in PBS to form the suspension.

In this context, the phrase "substantially no transmembrane potential" means a level of transmembrane potential below which a drug would not be actively loaded into the liposome. For example, see Akbarzadeh et al., Nanoscale Research Letters 2013, 8:102.

A hydrophobic drug is added to the suspension of liposomes in the aqueous solution to form a mixture. The hydrophobic drug can be sirolimus, umirolimus, or everolimus. In a particular method, the drug is umirolimus. The loading method at this stage does not require the use of a solubility enhancer, e.g. a cyclodextrin, to solubilize the hydrophobic drug in the aqueous solution. Indeed, this is not necessary or desirable.

Not to be bound by theory, it is believed that the hydrophobic drug, as it is added to the liposomes suspended in the aqueous solution, interact strongly with lipid tails of the liposomes due to the hydrophobic nature of the drug, leading to its encapsulation inside the lipid bilayer of the liposome.

The mixture of liposomes and hydrophobic drug is stirred for 4 to 48 hours at room temperature, resulting in at least 80% (e.g., 80%, 85%, 90%, 95%, and 100%) of the added hydrophobic drug loaded into the cholesterol-free liposomes.

Also within the scope of the application is a method for treating cancer using the stable liposomal formulations described above. The method requires administering to a cancer patient an effective amount of the stable liposomal formulation that contains an anti-cancer drug selected from sirolimus, umirolimus, or everolimus. The effective amount inhibits growth of cancer cells in the patient.

A skilled person in the art can readily determine the effective amount of the stable liposomal formulation that should be administered to the cancer patient. For example, response to drug dose over time can be followed by measuring tumor size by MRI or CT scan.

The stable liposomal formulations can be administered to a cancer patient via any conventional method, including, but not limited to, intraperitoneal injection, intravenous injection, direct injection into a tumor, injection into the arterial circulation upstream of a tumor, and nasal inhalation. The stable liposomal formulations described above can also be formed into a pill or a capsule for oral administration.

The types of cancer that can be treated by administering the above-described stable liposomal formulations include but are not limited to acute lymphocytic leukemia, acute myeloid leukemia, adrenal cancer, adult soft tissue sarcoma, anal cancer, aplastic anemia, basal and squamous cell skin cancer, bile duct cancer, bladder cancer, bone cancer, brain/CNS tumors, breast cancer, breast cancer in man, cancer in children, cancer of unknown primary, Castleman's disease, cervical cancer, chronic lymphocytic leukemia, chronic myeloid leukemia, chronic myelomonocytic leukemia, colorectal cancer, endometrial cancer, esophagus cancer, Ewing Family of tumors, eye cancer, gallbladder cancer, gastric cancer, gastrointestinal carcinoid, gastrointestinal stromal tumor, gestational trophoblastic disease, Hodgkin's disease, Kaposi's sarcoma, kidney cancer, laryngeal and hypopharyngeal cancer, leukemia in children, liver cancer, lung cancer-non small cell, lung cancer-small cell, Lung carcinoid tumor, malignant mesothelioma, melanoma skin cancer, multiple myeloma, myelodysplastic syndrome, nasal cavity and paranasal sinus cancer, nasopharyngeal cancer, neuroblastoma, non-Hodgkin's lymphoma, oral cavity and oropharangeal cancer, osteosarcoma, ovarian cancer, pancreatic cancer, penile cancer, pituitary tumors, prostate cancer, renal cell carcinoma, retinoblastoma, rhabdomyosarcoma, salivary gland cancer, skin lymphoma, small intestine cancer, stomach cancer, testicular cancer, thymus cancer, thyroid cancer, uterine sarcoma, vaginal cancer, vulvar cancer, Waldenstrom's macroglobulinemia, and Wilms' tumor.

Without further elaboration, it is believed that one skilled in the art can, based on the disclosure herein, utilize the present invention to its fullest extent. The following specific examples are, therefore, to be construed as merely descriptive, and not limitative of the remainder of the disclosure in any way whatsoever.

### EXAMPLES

### EXAMPLE 1: Preparation of umirolimus-loaded liposomes

Four 200 mL batches of liposomes were prepared, each containing a different phosphatidylcholine, namely, EggPC, POPC, DMPC, or DOPC. Briefly, 6000 mg of each phosphatidylcholine was added to 200 mL of phosphate buffered saline (PBS) in separate 500 mL depyrogenated glass bottles. The mixtures were stirred at room temperature for at least 30 min. to form multilamellar vesicles (MLVs). The sizes of the MLVs were reduced by extrusion through a 3-stack of polycarbonate filter membranes (pore size 100 nm) using a bench top extruder (Northern Lipids Inc., Canada). After 10 extrusion passes, large unilamellar vesicles (LUVs) with an average size of -100 nm were obtained.

To load umirolimus into the LUVs, 50 mg of the drug was added to each of four 50 mL depyrogenated glass bottle together with 10 mL of one of the four LUV preparations per bottle. The glass bottle was capped and placed in a water bath at a temperature of 25°C. The mixture was stirred for 24 hours. The resulting solution of umirolimus-loaded liposomes was filtered through a polyvinylidene difluoride (PVDF) syringe filter having a 0.2 µm pore size to remove un-encapsulated umirolimus.

The umirolimus content of the liposomal solutions after loading was determined by reverse-phase HPLC. The liposomes were broken by mixing 50 µl samples of each liposomal solution with 1.0 ml of acetonitrile. A standard solution of umirolimus was prepared at 0.05 mg/mL in methanol. Samples were analyzed by HPLC and compared to the standard solution to quantify the amount of umirolimus in the liposome.

The intensity mean diameter of the liposomes and the polydispersity index (PDI) of the distribution were determined by dynamic light scattering (DLS) using a Zetasizer Nano instrument (Malvern Instruments, Great Britain). Each sample tested was diluted 1:25 in a 0.9% sodium chloride solution. Particle size measurements at a scattering angle of 172° were carried out using a scan of at least 5 min. with the following parameters: viscosity = 1.0183 cp, refractive index = 1.332, temperature = 23°C. The size of the liposomes were measured and recorded prior to and after umirolimus loading. The drug loading results are summarized in the Table 1 below.

**Table 1. Umirolimus Loading into Liposomes**

| Lipid type | EggPC | POPC | DOPC | DMPC |
|---|---|---|---|---|
| Vesicle size prior to drug loading (nm) | 98.0 | 96.6 | 97.6 | 88.6 |
| PDI prior to drug loading | 0.070 | 0.039 | 0.059 | 0.072 |
| Umirolimus content (mg/mL) | 1.71 | 2.76 | 2.72 | 2.98 |
| Vesicle size at end of drug loading (nm) | 102.0 | 99.0 | 99.0 | 91.8 |
| PDI at end of drug loading | 0.031 | 0.049 | 0.038 | 0.048 |
| Drug:Lipid ratio (w/w) | 1:17.5 | 1:10.9 | 1:11.0 | 1:10.1 |

The amount of umirolimus loaded into liposomes formed of DMPC, POPC, and DOPC was greater than 2.7 mg/mL. Unexpectedly, the amount of umirolimus loaded into these three liposomes was higher than that loaded into liposomes formed of EggPC.

### EXAMPLE 2: Preparation of umirolimus-loaded liposomes containing a polyethylene glycol-conjugated phospholipid

Liposomes containing a polyethylene glycol-conjugated (PEGylated) phospholipid were prepared by combining 1800 mg of POPC and 200 mg of DSPE PEG-2000 in 200 mL of PBS in a 500 mL depyrogenated glass bottle. The mixture was stirred at room temperature for at least 30 min. to form MLVs. The sizes of the MLVs were reduced by extrusion through a 3-stack of polycarbonate filter membranes (pore size 100 nm) using a bench top extruder (Northern Lipids Inc., Canada). After 10 extrusion passes, PEGylated LUVs with an average size of approximately 100 nm were obtained.

To load umirolimus into the PEGylated LUVs, 50 mg of the drug was added to a 500 mL depyrogenated glass bottle together with 50 mL of the LUVs. The glass bottle was capped and placed in a water bath at a temperature of 25°C. The mixture was stirred for up to 24 hours. 1 mL samples were collected at 1, 2, 3, 4, 5, 6, and 24 h after initiation of stirring to evaluate umirolimus loading into the PEGylated liposomes. Each sample was filtered through a polyvinylidene difluoride (PVDF) syringe filter having a 0.2 µm pore size to remove un-encapsulated umirolimus. The umirolimus content of each sample was evaluated by HPLC as described above in

### EXAMPLE 1.

The results indicated that the percentage of umirolimus incorporated into the PEGylated liposomes after stirring for 1, 2, 3, 4, 5, 6, and 24 h was 59%, 67%, 77%, 82%, 87%, 91%, and 102%, respectively, based on the initial amount of umirolimus used for loading. Performing the above-described loading method unexpectedly resulted in loading of essentially all of the umirolimus into the PEGylated liposomes within 24 h.

The stability of the umirolimus-loaded PEGylated liposome formulation was evaluated during storage in clear glass vials at 5°C ± 3°C. The sample temperature was monitored and recorded continuously to ensure constant temperature conditions. The solution was analyzed for umirolimus content, vesicle size, and PDI as described above in EXAMPLE 1 after storage for 2, 3, 4, 6, and 8 weeks. The stability results are shown in TABLE 2 below.

**TABLE 2. Stability of formulations of umirolimus-loaded PEGylated liposomes**

| Time Point | 0 weeks (end of drug loading) | 2 week | 3 week | 4 week | 6 week | 8 week |
|---|---|---|---|---|---|---|
| umirolimus content (mg/mL) | 0.99 | 1.00 | 1.00 | 0.96 | 1.0 | 1.0 |
| vesicle size (nm) | 99.9 | 97.1 | 97.5 | 97.5 | 97.7 | 98.3 |
| PDI | 0.062 | 0.048 | 0.058 | 0.053 | 0.050 | 0.048 |

The results indicate that the solution of umirolimus-loaded PEGylated liposomes is stable for at least 8 weeks when stored at 5°C.

### EXAMPLE 3: Measurement of drug encapsulation efficiency of umirolimus-loaded liposome formulations

An assay was developed to characterize the efficiency of umirolimus encapsulation by the liposomes using a gel-filtration technique to remove free drug from the liposome solution. The drug to lipid ratio of liposomal formulations was determined before and after running them on a PD-10 cross-linked dextran gel (SEPHADEX® G-25) desalting column using the following equation: $Drug Encapsulation % = \frac{Final Drug : Lipid Ratio \left(samples passed through PD-10 column\right)}{Initial Drug : Lipid Ratio \left(samples prior to PD-10 separation\right)} \times 100 %$

Umirolimus-loaded POPC liposomes and umirolimus-loaded POPC PEGylated liposomes were prepared as described above in EXAMPLE 1 and EXAMPLE 2, respectively. The drug encapsulation efficiencies are shown in TABLE 3 below.

**TABLE 3. Umirolimus drug encapsulation efficiency**

| Formulation | Drug:Lipid ratio prior to column separation | Drug:Lipid ratio after column separation | Drug encapsulation efficiency (%) |
|---|---|---|---|
| g-loaded POPC liposomes from SAMPLE 1 (drug:lipid = 1:10) | 0.130 | 0.125 | 95.9 |
| Drug-loaded PEGylated OSPE/POPC liposomes from KAMPLE 2 (drug:lipid = 1:20) | 0.052 | 0.050 | 97.5 |

Surprisingly, the encapsulation efficiency of both the umirolimus-loaded POPC liposomes and the umirolimus-loaded POPC PEGylated liposomes was greater than 95%.

### EXAMPLE 4: Preparation of sirolimus- and everolimus-loaded liposomes

POPC LUVs were prepared as described above in EXAMPLE 1. 50 mg of sirolimus or everolimus were added to a 50 mL depyrogenated glass bottle together with 10 mL of the POPC LUVs. The glass bottle was capped and placed in a water bath at a temperature of 25°C. The mixture was stirred at room temperature for 24 h. The drug-loaded liposome solution was filtered through a 0.2µm PVDF syringe filter to removed un-encapsulated drug.

For both liposomal formulations, the drug content after encapsulation, the intensity mean diameter of the liposomes, and the PDI were determined as described above in EXAMPLE 1. The results are shown in Table 4 below.

**Table 4. Drug-Loading of sirolimus and everolimus into POPC liposomes**

| Drug | Sirolimus | Everolimus |
|---|---|---|
| Vesicle Size prior to Drug Loading (nm) | 96.6 | 96.6 |
| PDI prior to Drug Loading | 0.039 | 0.039 |
| Drug content (mg/mL) | 1.85 | 4.65 |
| Drug:Lipid Ratio (w/w) | 1:16 | 1:6.5 |
| Vesicle Size at end of Drug Loading (nm) | 96.1 | 99.5 |
| PDI at end of Drug Loading | 0.048 | 0.087 |
| Drug Encapsulation Efficiency (%) | 95.7 | 98.7 |

The results indicated that sirolimus and everolimus were successfully encapsulated into POPC liposomes at high efficiencies.

### EXAMPLE 5: In vitro drug release study

An *in vitro* drug release assay was used to determine release profiles of umirolimus, sirolimus, and everolimus. Umirolimus-loaded liposomes were prepared as described above in EXAMPLE 1, and sirolimus-loaded and everolimus-loaded liposomes were prepared as described above in EXAMPLE 4.

For each drug, a control formulation was prepared containing 1.0 mg of drug in 5 mL of a solution containing 15% acetonitrile and 85% water. 5 mL of the control formulations and the liposomal formulations for each drug were loaded into separate dialysis tubes having a molecular weight cut-off of 50 kDal.

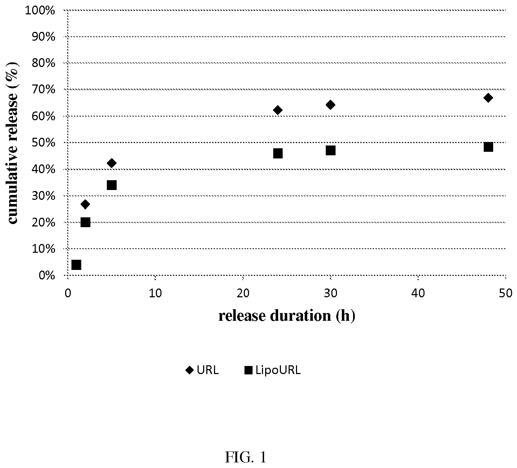

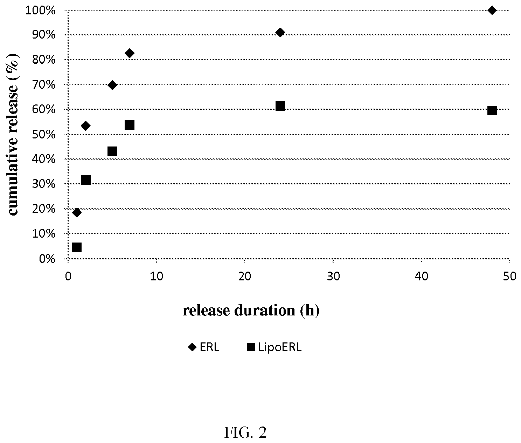

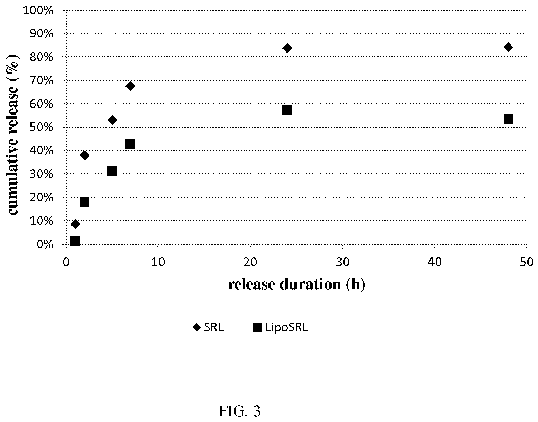

Each loaded dialysis tube was placed in an individual 50 mL tube containing 40 mL of release media (15% acetonitrile and 0.5 % SDS). The release media in each tube was sampled after 1, 2, 5, 7, 24, 30, and 48 h and the drug concentration in the release media was determined by HPLC as described above in EXAMPLE 1. The cumulative drug release percentage versus release time was plotted for all samples. The results are shown in Figures 1, 2, and 3 for umirolimus, everolimus, and sirolimus, respectively.

The results indicated that, respectively, 67%, 100%, and 84% of the starting amounts of umirolimus, everolimus, and sirolimus diffused out of the dialysis tubing within 48 h.

By contrast, the liposome-encapsulated drugs diffused out of the dialysis tubing at a slower rate. The cumulative release of encapsulated umirolimus, everolimus, and sirolimus after 48 h was 67%, 59%, and 54%, respectively, of the starting amount of drug.

The results indicated that liposomal formulations of umirolimus, everolimus, and sirolimus are effective for sustained drug delivery.

## Claims

1. A stable liposomal formulation, the stable formulation comprising a liposome that contains at least one lipid bilayer formed of a phosphatidylcholine selected from the group consisting of palmitoyloleoylphosphatidylcholine (POPC), dimyristoylphosphatidylcholine (DMPC), and dioleoylphosphatidylcholine (DOPC) or mixtures thereof; and a drug encapsulated in the liposome, wherein the drug is sirolimus, umirolimus, or everolimus and the liposome has a diameter of 50 nm to 2 µm and is free of cholesterol.

2. The stable liposomal formulation of claim 1, wherein the at least one lipid bilayer further includes a phospholipid conjugated to a polyethylene glycol (PEG) moiety.

3. The stable liposomal formulation of claim 2, wherein the phospholipid is distearoylphosphatidylethanolamine (DSPE) and the PEG moiety has a molecular weight of 150 to 3000 g/mol.

4. The stable liposomal formulation of any of claims 1 to 3, wherein a weight ratio between the drug and the phosphatidylcholine is 1:5 to 1:100.

5. The stable liposomal formulation of claim 4, wherein the formulation contains 0.01 mg/mL to 10 mg/mL of the drug.

6. The stable liposomal formulation of any of claims 1 to 5, wherein the formulation has a pH of 6 to 8.

7. The stable liposomal formulation of claim 6, wherein the phosphatidylcholine is POPC, the drug is umirolimus, the weight ratio between the umirolimus and the phosphatidylcholine is 1:20, and the formulation contains 1 mg/mL of the umirolimus.

8. A method for loading a hydrophobic drug into liposomes, the method comprising:
obtaining cholesterol-free liposomes having at least one lipid bilayer,
adding the cholesterol-free liposomes to an aqueous solution to form a suspension such that there is substantially no transmembrane potential across the at least one lipid bilayer,
adding a hydrophobic drug in the absence of a solubility enhancer to the suspension to form a mixture, and
stirring the mixture for 4 to 48 hours at room temperature,
whereby at least 80% of the added hydrophobic drug is loaded into the cholesterol-free liposome, wherein the hydrophobic drug is sirolimus, umirolimus, or everolimus and wherein the cholesterol-free liposomes have a diameter of 80 nm to 2 µm and are obtained by forming multilamellar vesicles (MLVs) that contain one or more of palmitoyloleoylphosphatidylcholine (POPC), dimyristoylphosphatidylcholine (DMPC), dioleoylphosphatidylcholine (DOPC); and extruding the MLVs, thereby obtaining the cholesterol-free liposomes having a diameter of 50 nm to 2 µm.

9. The method of claim 8, wherein the MLVs also contain polyethylene glycol-conjugated distearoylphosphatidylethanolamine (DSPE-PEG).

10. A method for preparing a hydrophobic drug encapsulated in a cholesterol-free liposome, the method comprising:
suspending one or more of palmitoyloleoylphosphatidylcholine (POPC), dimyristoylphosphatidylcholine (DMPC), and dioleoylphosphatidylcholine (DOPC) in an aqueous buffer to form a lipid suspension,
stirring the lipid suspension for at least 30 minutes at room temperature to form multilamellar vesicles (MLVs),
extruding the MLVs to form large unilamellar vesicles (LUVs) having a diameter of 50 nm to 2 µm,
adding the LUVs to an aqueous solution to form a suspension such that there is substantially no transmembrane potential across the LUVs,
adding a hydrophobic drug to the suspension in the absence of a solubility enhancer to form a mixture,
stirring the mixture for 4 to 48 hours at room temperature to form a drug-loaded liposome suspension, and
filtering the drug-loaded liposome suspension to remove unencapsulated hydrophobic drug,
whereby at least 80% of the added hydrophobic drug is encapsulated in the cholesterol-free liposomes, wherein the hydrophobic drug is sirolimus, umirolimus, or everolimus.

11. The method of claim 10, wherein polyethylene glycol-conjugated distearoylphosphatidylethanolamine (DSPE-PEG) is also suspended in the aqueous buffer in the suspending step.

12. The stable liposomal formulation of any one of claims 1-7 for use in treating cancer, preferably wherein the stable liposomal formulation inhibits growth of cancer cells.

## Patentansprüche

1. Eine stabile liposomale Formulierung, wobei die stabile Formulierung ein Liposom umfasst, das wenigstens eine Lipiddoppelschicht, gebildet aus einem Phosphatidylcholin, ausgewählt aus der Gruppe, bestehend aus Palmitoyloleoylphosphatidylcholin (POPC), Dimyristoylphosphatidylcholin (DMPC) und Dioleoylphosphatidylcholin (DOPC) oder Gemischen davon, und ein Medikament, verkapselt in dem Liposom, enthält, wobei das Medikament Sirolimus, Umirolimus oder Everolimus ist und das Liposom einen Durchmesser von 50 nm bis 2 µm besitzt und frei von Cholesterol ist.

2. Die stabile liposomale Formulierung nach Anspruch 1, wobei wenigstens eine Lipiddoppelschicht ein Phospholipid enthält, das an eine Polyethylenglycol-(PEG)-Komponente konjugiert ist.

3. Die stabile liposomale Formulierung nach Anspruch 2, wobei das Phospholipid Distearoylphosphatidylethanolamin (DSPE) ist und die PEG-Komponente ein Molekulargewicht von 150 bis 3000 g/mol besitzt.

4. Die stabile liposomale Formulierung nach einem der Ansprüche 1 bis 3, wobei ein Gewichtsverhältnis zwischen dem Medikament und dem Phosphatidylcholin 1:5 bis 1:100 beträgt

5. Die stabile liposomale Formulierung nach Anspruch 4, wobei die Formulierung 0,01 mg/ml bis 10 mg/ml Medikament enthält.

6. Die stabile liposomale Formulierung nach einem der Ansprüche 1 bis 5, wobei die Formulierung einen pH von 6 bis 8 besitzt.

7. Die stabile liposomale Formulierung nach Anspruch 6, wobei das Phosphatidylcholin POPC ist, das Medikament Umirolimus ist, das Gewichtsverhältnis zwischen Umirolimus und dem Phosphatidylcholin 1:20 beträgt und die Formulierung 1 mg/ml Umirolimus enthält.

8. Ein Verfahren zum Laden eines hydrophoben Medikaments in Liposomen, wobei das Verfahren umfasst:
Gewinnen Cholesterol-freier Liposomen mit wenigstens einer Lipiddoppelschicht,
Zugabe der Cholesterol-freien Liposomen zu einer wässrigen Lösung, um eine Suspension zu bilden, so dass es im Wesentlichen kein Transmembranpotential über die wenigstens eine Lipiddoppelschicht entsteht,
Zugabe eines hydrophoben Medikaments in Abwesenheit eines Löslichkeitsverstärkers zu der Suspension, um ein Gemisch zu bilden,
Rühren des Gemisches 4 bis 48 Stunden bei Raumtemperatur,
wodurch wenigstens 80% des zugegebenen Medikaments in das Cholesterol-freie Liposom geladen werden, wobei das hydrophobe Medikament Sirolimus, Umirolimus oder Everolimus ist und wobei die Cholesterol-freien Liposomen einen Durchmesser von 80 nm bis 2 µm besitzen und durch Bilden multilamellarer Vesikel (MLVs), die eines oder mehrere aus Palmitoyloleoylphosphatidylcholin (POPC), Dimyristoylphosphatidylcholin (DMPC) und Dioleoylphosphatidylcholin (DOPC) enthalten, und Extrudieren der MLVs erhalten werden, wobei dadurch die Cholesterol-freien Liposomen mit einem Durchmesser von 50 nm bis 2 µm erhalten werden.

9. Das Verfahren nach Anspruch 8, wobei die MLVs ebenfalls Polyethylenglycol-konjugiertes Distearoylphosphatidylethanolamin (DSPE-PEG) enthalten.

10. Ein Verfahren zur Herstellung eines hydrophoben Medikaments, verkapselt in einem Cholesterol-freien Liposom, wobei das Verfahren umfasst:
Suspendieren eines oder mehrerer aus Palmitoyloleoylphosphatidylcholin (POPC), Dimyristoylphosphatidylcholin (DMPC) und Dioleoylphosphatidylcholin (DOPC) in einem wässrigen Puffer, um eine Lipidsuspension zu bilden,
Rühren der Lipidsuspension wenigstens 30 Minuten bei Raumtemperatur, um multilamellare Vesikel (MLVs) zu bilden,
Extrudieren der MLVs, um große unilamellare Vesikel (LUVs) mit einem Durchmesser von 50 nm bis 2 µm zu bilden,
Zugabe der LUVs zu einer wässrigen Lösung, um eine Suspension zu bilden, so dass im Wesentlichen kein Transmembranpotential über die LUVs entsteht,
Zugabe eines hydrophoben Medikaments zu der Suspension in Abwesenheit eines Löslichkeitsverstärkers, um ein Gemisch zu bilden, und
Rühren des Gemisches 4 bis 48 Stunden bei Raumtemperatur, um eine Suspension von Medikament-beladenen Liposomen zu bilden,
Filtern der Suspension von Medikament-beladenen Liposomen, um nichtverkapseltes hydrophobes Medikament zu entfernen,
wodurch wenigstens 80% des zugegebenen hydrophoben Medikaments in die Cholesterol-freien Liposomen geladen werden, wobei das hydrophobe Medikament Sirolimus, Umirolimus oder Everolimus ist.

11. Das Verfahren nach Anspruch 10, wobei Polyethylenglycol-konjugiertes Distearoylphosphatidylethanolamin (DSPE-PEG) ebenfalls in dem wässrigen Puffer in dem Suspendierungsschritt suspendiert wird.

12. Die stabile liposomale Formulierung nach einem der Ansprüche 1 bis 7 zur Verwendung in der Behandlung von Krebs, vorzugsweise wobei die stabile liposomale Formulierung Wachstum von Krebszellen hemmt.

## Revendications

1. Formulation liposomiale stable, la formulation stable comprenant un liposome qui contient au moins une bicouche lipidique formée d'une phosphatidylcholine choisie dans le groupe constitué par la palmitoyl-oléoyl-phosphatidylcholine (POPC), la dimyristoyl-phosphatidylcholine (DMPC), et la dioléoyl-phosphatidylcholine (DOPC), ou leurs mélanges ; et un médicament encapsulé dans le liposome, dans laquelle le médicament est le sirolimus, l'umirolimus, ou l'évérolimus et le liposome a un diamètre de 50 nm à 2 µm et est exempt de cholestérol.

2. Formulation liposomiale stable selon la revendication 1, dans laquelle l'au moins une bicouche lipidique contient en outre un phospholipide conjugué à un fragment polyéthylèneglycol (PEG).

3. Formulation liposomiale stable selon la revendication 2, dans laquelle le phospholipide est la distéaroyl-phosphatidyléthanolamine (DSPE) et le fragment PEG a une masse moléculaire de 150 à 3 000 g/mol.

4. Formulation liposomiale stable selon l'une quelconque des revendications 1 à 3, dans laquelle le rapport en poids entre le médicament et la phosphatidylcholine est de 1/5 à 1/100.

5. Formulation liposomiale stable selon la revendication 4, laquelle formulation contient 0,01 mg/ml à 10 mg/ml du médicament.

6. Formulation liposomiale stable selon l'une quelconque des revendications 1 à 5, laquelle formulation a un pH de 6 à 8.

7. Formulation liposomiale stable selon la revendication 6, dans laquelle la phosphatidylcholine est la POPC, le médicament est l'umirolimus, le rapport en poids entre l'umirolimus et la phosphatidylcholine est de 1/20, et la formulation contient 1 mg/ml de l'umirolimus.

8. Méthode pour charger un médicament hydrophobe dans des liposomes, la méthode comprenant :
l'obtention de liposomes exempts de cholestérol ayant au moins une bicouche lipidique,
l'addition des liposomes exempts de cholestérol à une solution aqueuse pour former une suspension de façon qu'il n'y ait pratiquement pas de potentiel transmembranaire à travers l'au moins une bicouche lipidique,
l'addition d'un médicament hydrophobe en l'absence d'un amplificateur de solubilité à la suspension pour former un mélange, et
l'agitation du mélange pendant 4 à 48 heures à la température ambiante,
moyennant quoi au moins 80 % du médicament hydrophobe ajouté sont chargés dans le liposome exempt de cholestérol, dans laquelle le médicament hydrophobe est le sirolimus, l'umirolimus, ou l'évérolimus, et dans laquelle les liposomes exempts de cholestérol ont un diamètre de 80 nm à 2 µm et sont obtenus par formation de vésicules multilamellaires (MLV) qui contiennent une ou plusieurs parmi la palmitoyl-oléoyl-phosphatidylcholine (POPC), la dimyristoyl-phosphatidylcholine (DMPC), et la dioléoyl-phosphatidylcholine (DOPC) ; et l'extrusion des MLV, ce qui donne ainsi les liposomes exempts de cholestérol ayant un diamètre de 50 nm à 2 µm.

9. Méthode selon la revendication 8, dans laquelle les MLV contiennent aussi de la distéaroyl-phosphatidyléthanolamine conjuguée à du polyéthylèneglycol (DSPE-PEG).

10. Méthode pour préparer un médicament hydrophobe encapsulé dans un liposome exempt de cholestérol, la méthode comprenant :
la mise en suspension d'une ou plusieurs parmi la palmitoyl-oléoyl-phosphatidylcholine (POPC), la dimyristoyl-phosphatidylcholine (DMPC), et la dioléoyl-phosphatidylcholine (DOPC) dans un tampon aqueux pour former une suspension de lipides,
l'agitation de la suspension de lipides pendant au moins 30 minutes à la température ambiante pour former des vésicules multilamellaires (MLV),
l'extrusion des MLV pour former de grosses vésicules unilamellaires (LUV) ayant un diamètre de 50 nm à 2 µm,
l'addition des LUV à une solution aqueuse pour former une suspension de façon qu'il n'y ait pratiquement pas de potentiel transmembranaire à travers les LUV,
l'addition d'un médicament hydrophobe à la suspension en l'absence d'un amplificateur de solubilité pour former un mélange,
l'agitation du mélange pendant 4 à 48 heures à la température ambiante pour former une suspension de liposomes chargés de médicament, et
la filtration de la suspension de liposomes chargés de médicament pour éliminer le médicament hydrophobe non encapsulé,
moyennant quoi au moins 80 % du médicament hydrophobe ajouté sont encapsulés dans les liposomes exempts de cholestérol, dans laquelle le médicament hydrophobe est le sirolimus, l'umirolimus, ou l'évérolimus.

11. Méthode selon la revendication 10, dans laquelle de la distéaroyl-phosphatidyléthanolamine conjuguée à du polyéthylèneglycol (DSPE-PEG) est aussi mise en suspension dans le tampon aqueux dans l'étape de mise en suspension.

12. Formulation liposomiale stable selon l'une quelconque des revendications 1 à 7, pour une utilisation dans le traitement d'un cancer, de préférence laquelle formulation liposomiale stable inhibe la croissance de cellules cancéreuses.
